# EUROPÄISCHE PATENTSCHRIFT

(11) **EP 4 231 827 B1**
(45) Veröffentlichungstag und Bekanntmachung des Hinweises auf die Patenterteilung: **20.05.2026**
(21) Anmeldenummer: 21791272.4
(22) Anmeldetag: 05.10.2021
(51) Int. Cl.: A01N 1/142, A01N 1/16, C12N 5/00

(54) **VERFAHREN UND ENTSPRECHENDE VORRICHTUNG ZUR KRYOKONSERVIERUNG EINER VIELZAHL VON ZELLVERBÄNDEN AUS BIOLOGISCHEN ZELLEN**
METHOD AND CORRESPONDING DEVICE FOR CRYOPRESERVING A PLURALITY OF CELL CLUSTERS OF BIOLOGICAL CELLS
PROCÉDÉ ET DISPOSITIF CORRESPONDANT DE CRYOCONSERVATION D'UNE PLURALITÉ DE GROUPES CELLULAIRES COMPOSÉS DE CELLULES BIOLOGIQUES

(30) Priorität: 22.10.2020 DE 102020127787
(43) Veröffentlichungstag der Anmeldung: 30.08.2023
(73) Patentinhaber: Fraunhofer-Gesellschaft zur Förderung der angewandten Forschung e.V., 80686 München (DE)
(72) Erfinder: ZIMMERMANN, Heiko, 66280 Sulzbach (DE); MEISER, Ina, 66280 Sulzbach (DE); STRACKE, Frank, 66280 Sulzbach (DE)
(74) Vertreter: v. Bezold & Partner Patentanwälte - PartG mbB
(86) Internationale Anmeldenummer: PCT/EP2021/077425
(87) Internationale Veröffentlichungsnummer: WO 2022/084025

(56) Entgegenhaltungen:
- WO-A1-2016/064896
- WO-A2-2014/145075
- WU MENGXI ET AL: "Acoustofluidic separation of cells and particles", MICROSYSTEMS & NANOENGINEERING, vol. 5, no. 1, 1 December 2019 (2019-12-01), pages 32, XP055884657, DOI: 10.1038/s41378-019-0064-3

## Beschreibung

Die Erfindung betrifft ein Verfahren und eine Kryokonservierungsvorrichtung zur Kryokonservierung einer Vielzahl von Zellverbänden aus biologischen Zellen (auch als Zellaggregate bezeichnet), z. B. von Zellgewebe oder Organoiden. Anwendungen der Erfindung sind z. B. in der Biomedizin und/oder Biotechnologie gegeben.

In der biotechnologischen/pharmakologischen Forschung und in der Biomedizin, wie z. B. der Transplantationsmedizin, besteht ein Interesse an Anwendungen von Zellverbänden aus einer Vielzahl biologischer Zellen. Zellverbände bieten insbesondere die Fähigkeit, spezifische Eigenschaften oder Funktionen von Organen eines biologischen Organismus nachzubilden, ohne dass sie ein vollständiges Organ bilden. Zellverbände umfassen z. B. biologische native Gewebe (in vivo gewachsene Zell-Matrix-Verbände), Sphäroide (kugelförmige Zellhaufen) oder Organoide (künstlich in vitro gezüchtete Zell-Matrix-Verbände). Die Bildung von Zellverbänden kann Kultivierungsdauern von Tagen, Wochen oder sogar Monaten erfordern, wobei sich einzelne Zellverbände verschieden schnell entwickeln. Im Ergebnis eines Kultivierungsverfahrens werden daher typischerweise inhomogene Proben mit Zellverbänden in verschiedenen Reife- oder Entwicklungsstadien, insbesondere Größen, erzeugt.

Die Kryokonservierung biologischer Materialien, wie z. B. Zellen, Zellbestandteile und/oder Zellgruppen, ist ein allgemein bekanntes Verfahren, mit dem biologische Materialien vitalitätserhaltend eingefroren werden. Das Einfrieren erfolgt gemäß vorbestimmten Einfrierprotokollen, typischerweise unter Zusatz eines Gefrierschutzmittels (Kryoprotektivum, CPA), mit dem die Bildung von Eiskristallen beim Einfrieren verhindert oder unterdrückt wird. Verfahrensparameter (Bedingungen einer Vorbehandlung und eines nachfolgenden Einfrierens) werden insbesondere in Abhängigkeit von den Eigenschaften der biologischen Materialien gewählt (siehe z. B. Übersichtsartikel von M. A. Taylor et al. "New Approaches to Cryopreservation of Cells, Tissues and Organs" in "Transfus. Med. Hemother." 46: 2019, 197-215).

Für die effektive Kryokonservierung von Suspensionen aus einzelnen Zellen mit einer hohen Vitalitätsausbeute werden Zelltyp-spezifische Verfahrensparameter, wie z. B. Hilfsstoff-Zusammensetzung, Einwirkungsdauer und Kühlgeschwindigkeit optimiert, was einen großen Aufwand bedeuten und erhebliche Erfahrungen erfordern kann. Bei der Kryokonservierung von Zellverbänden ist der Konservierungserfolg noch empfindlicher von der Wahl der Verfahrensparameter abhängig, so dass auch der Aufwand der Wahl optimierter Einfrierparameter wächst.

Während die Kryokonservierung von einzelnen Zellen in Suspension insbesondere durch langsames Einfrieren verbreitet angewendet wird, können inhomogene Proben aus ausgedehnten, dreidimensionalen Zellverbänden, wie z. B. Gewebe, Sphäroide oder Organoide, mit verschiedenen Größen durch langsames Einfrieren bisher nur mit beschränkter Ausbeute eingefroren werden. Beispielsweise steigt die erforderliche Einwirkungsdauer von Hilfsstoffen, die zu einer gewünschten Konzentration im Inneren des Zellverbandes führt, quadratisch mit dessen Durchmesser. Da bei üblichen (skalierbaren) Produktionsverfahren von Zellverbänden typischerweise die oben genannte Inhomogenität, insbesondere eine Polydispersität, auftritt, wären auch bei einem in Bezug auf den Zelltyp und eine bestimmte Größe optimal gewählten Konservierungsprotokoll Verluste durch die für abweichende Größen unpassenden Einwirkungsdauern und -bedingungen zu erwarten. Bei der herkömmlichen Kryokonservierung einer polydispersen Probe von Zellverbänden müssen daher bisher immer Kompromisse bei der Wahl der Einfrierparameter akzeptiert werden.

Es ist auch bekannt, kleinere Zellverbände durch Vitrifikation (Verglasung durch ultraschnelles Kühlen) zu konservieren. Zur Bereitstellung von sehr hohen Konzentrationen von Hilfsstoffen und zum ultraschnellen Erreichen von Temperaturen unterhalb des Glaspunktes an jedem Ort des dreidimensionalen Gewebeverbandes sind der Vitrifikation jedoch enge technische Grenzen gesetzt. Dies betrifft vor allem das Volumen der Probe, das durch die begrenzte Wärmeleitung wässriger Medien nach oben limitiert ist (λ = 0,56 W/Km, α = 0,14 mm²/s). Jedoch auch die Hilfsstoffkonzentrationen und Einwirkdauern der Hilfsstoffe sind aufgrund von deren Zytotoxizität begrenzt. Des Weiteren steigt die Wahrscheinlichkeit von Schäden durch thermische Spannungsrisse mit wachsender Probengröße. Für die Routinekonservierung von Proben mit vielen Zellverbänden verschiedener Größen ist die Vitrifikation daher ungeeignet.

Die genannten Beschränkungen bei der Wahl von Einfrierparametern treten in der Praxis nicht nur bei der Kryokonservierung von Proben mit Zellverbänden verschiedener Größen auf. Die Inhomogenität einer Probe kann sich auch aus dem Auftreten von anderen verschiedenen Eigenschaften, wie beispielsweise verschiedenen Formen oder Elastizitäten, gemeinsam bereitgestellter Zellverbände ergeben.

WO 2016/064896 A1 offenbart ein Verarbeitungsverfahren für eine Gewebeprobe sowie eine entsprechende Vorrichtung, welche ein mikrofluidische Fraktionierungseinrichtung und eine Kryokonservierungsvorrichtung umfasst. In der Fraktionierungseinrichtung werden Materialien in der Gewebeprobe beispielsweise nach Größe in verschiedene Fraktionen aufgeteilt und in entsprechenden Behältern gesammelt.

Die Aufgabe der Erfindung ist es, ein verbessertes Verfahren und eine verbesserte Kryokonservierungsvorrichtung zur Kryokonservierung einer Vielzahl von Zellverbänden aus biologischen Zellen bereitzustellen, mit denen Nachteile herkömmlicher Techniken vermieden werden. Die Kryokonservierung von Zellverbänden soll insbesondere in Bezug auf die Optimierung von Verfahrensparametern, die Ausbeute, die Effektivität und/oder die Anwendbarkeit bei verschiedenen Zelltypen verbessert werden.

Diese Aufgabe wird durch ein Verfahren und eine Kryokonservierungsvorrichtung zur Kryokonservierung einer Vielzahl von Zellverbänden aus biologischen Zellen mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die oben genannte Aufgabe durch ein Verfahren zur Kryokonservierung gemäß Anspruch 1 gelöst.

Gemäß einem zweiten allgemeinen Gesichtspunkt der Erfindung wird die oben genannte Aufgabe durch eine Kryokonservierungsvorrichtung gemäß Anspruch 7 gelöst.

Vorzugsweise ist die Kryokonservierungsvorrichtung oder eine ihrer Ausführungsformen zur Ausführung des Verfahren zur Kryokonservierung gemäß dem ersten allgemeinen Gesichtspunkt der Erfindung oder einer Ausführungsform des Verfahrens eingerichtet.

Erfindungsgemäß ist die Fraktionierung der Zellverbände in Abhängigkeit von mindestens einer vorbestimmten Eigenschaft der Zellverbände in mindestens zwei Fraktionen vorgesehen. Typischerweise werden bis zu 5 oder bis zu 10 Fraktionen gebildet. Es können aber auch mehr, z. B. bis zu 20 oder mehr Fraktionen vorgesehen sein.

Vorteilhafterweise werden mit der Fraktionierung der Zellverbände mindestens zwei homogene Fraktionen geschaffen, für die jeweils Verfahrensparameter der Kryokonservierung optimierbar sind. Die Erfinder haben festgestellt, dass optimale Verfahrensparameter der Kryokonservierung nicht nur vom Zelltyp abhängig sind, sondern auch von Eigenschaften eines Zellverbands an sich, wie z. B. der Größe des Zellverbands. Die Erfinder haben des Weiteren festgestellt, dass sich bei herkömmlichen Verfahren verschiedene Größen von Zellverbänden innerhalb einer inhomogenen Fraktion so auswirken, dass sich bei jeder Größe Hilfsstoffe und Wasser im Zellverband verschieden verteilen und damit das Einfrieren verschieden beeinflussen, was sich auf den Erfolg und die Ausbeute der Kryokonservierung nachteilig auswirkt. Durch die erfindungsgemäße Schaffung homogener Fraktionen werden die Beschränkungen der herkömmlichen Bearbeitung von inhomogenen Fraktionen überwunden.

Die Bereitstellung homogener Fraktionen hat einen weiteren Vorteil für Anwendungen von Zellverbänden, z. B. für Forschungszwecke oder für eine Implantationsbehandlung, bei denen ein Interesse an Fraktionen mit Zellverbänden in gleichen oder ähnlichen Reife- oder Entwicklungsstadien besteht. Derartige Fraktion können gebildet werden, indem gleiche Zellverbände für eine gewünschte Aufgabe durch ein Kultivierungsverfahren gewonnen, einer Kryokonservierung unterzogen und im gefrorenen Zustand gelagert werden.

Mit dem Begriff "Zellverband" wird eine zusammenhängende, vorzugsweise dreidimensional ausgedehnte Zellgruppe aus lebenden biologischen Zellen, wie z. B. ein Gewebe (insbesondere Gewebemodell), ein Sphäroid oder ein Organoid bezeichnet. Der Zellverband kann ausschließlich aus Zellen bestehen oder zusätzlich zu den Zellen auch extrazelluläre Matrixsubstanzen enthalten. Zellverbände werden z. B. durch eine Kultivierung biologischer Zellen und/oder durch eine Extraktion aus einem Organismus bereitgestellt. Mit dem Begriff "Fraktion" wird eine Vielzahl von Zellverbänden in einem flüssigen Umgebungsmedium bezeichnet.

Die Fraktionierung umfasst eine Auftrennung (Sortierung, Trennverfahren) von Zellverbänden aus einer zunächst inhomogenen Probe in eine vorbestimmte Zahl von Fraktionen. Die Auftrennung erfolgt derart, dass jede der Fraktionen Zellverbände enthält und die Zellverbände in jeder der Fraktionen mindestens eine gleiche Eigenschaft aufweisen. Dies bedeutet, dass die Zellverbände in jeder der Fraktionen in Bezug auf die mindestens eine Eigenschaft identisch sind oder so geringe Unterschiede haben, dass diese sich auf die Kryokonservierung, insbesondere die Wahl optimaler Parameter des Vorbehandlungsverfahrens und des Einfrierverfahrens, nicht auswirken. Jede der Fraktionen ist in Bezug auf die mindestens eine betrachtete Eigenschaft homogen. Die Fraktionierung ist insbesondere ein Trennverfahren, welches die Zellverbände vorzugsweise nicht verändert. Insbesondere bleiben die Zellverbände bei der Fraktionierung erhalten, d. h. die Zellverbände werden nicht in Teile zerlegt.

Die Fraktionierungseinrichtung umfasst vorzugsweise eine Trenneinrichtung, die zur Aufnahme einer Zusammensetzung der Zellverbände in einem Umgebungsmedium, zur Trennung der Zellverbände in die verschiedenen Fraktionen und zur Abgabe der Fraktionen in die verschiedenen Behälter eingerichtet ist.

Erfindungsgemäß werden die Fraktionen in verschiedenen Behältern gesammelt, d. h. die Fraktionierung umfasst eine Auftrennung in verschiedene Behälter. Jeder Behälter umfasst allgemein eine Aufnahme der Fraktion, die typischerweise die gleichen Zellverbände und ein flüssiges Umgebungsmedium, wie z. B. ein Nährmedium umfasst. Die Aufnahmen der verschiedenen Behälter sind voneinander getrennt.

Vorzugsweise werden die Fraktionen in den Behältern gesammelt, in denen nachfolgend die Kryokonservierung erfolgt. Vorteilhafterweise werden damit das Verfahren aus Fraktionierung und Kryokonservierung und der Aufbau der Kryokonservierungsvorrichtung vereinfacht und nach der Fraktionierung etwaige unerwünschte Einflüsse auf die Zellverbände vermieden.

Bei der Kryokonservierung werden die getrennten Fraktionen, wie sie bei der Fraktionierung gewonnen werden, eingefroren. Alternativ werden die Fraktionen vor dem Einfrieren einem Austausch des Umgebungsmediums und/oder einer Anreicherung der Zellverbände im Umgebungsmedium unterzogen.

Die Kryokonservierung der Zellverbände umfasst ein Vorbehandlungsverfahren und nachfolgend ein Einfrierverfahren. Die Vorbehandlung (oder: Inkubation) der Zellverbände umfasst eine Vorbereitung der Zellverbände für das Einfrieren, wobei z. B. die Zusammensetzung des flüssigen Umgebungsmediums mit Hilfsstoffen oder CPA (Gesamtheit aller für die Verbesserung des Konservierungsergebnis eingesetzten Zusätze), das Volumen der Fraktion, die Dichte der Zellverbände in der Fraktion und/oder weitere Vorbehandlungsparameter eingestellt und/oder die Zellverbände mit physikalischen und/oder chemischen Verfahren verändert, z. B. permeabilisiert, werden. Die Vorbehandlung der Zellverbände erfolgt vorzugsweise bei einer Temperatur, bei der das Umgebungsmedium flüssig ist, insbesondere bei Raumtemperatur. Das Einfrieren umfasst die Temperaturabsenkung der Fraktion unter 0°C bis zu einer Kryokonservierungstemperatur, z. B. im Bereich von - 80 °C bis - 200 °C. Einfrierparameter des Einfrierens sind z. B. der Zeitverlauf der Temperaturabsenkung und die eingestellte Kryokonservierungstemperatur.

Die Vorbehandlungsverfahren und Einfrierverfahren der Kryokonservierung werden in an sich bekannter Weise ausgeführt, wobei jedoch erfindungsgemäß bei der Kryokonservierung der Zellverbände der mindestens zwei Fraktionen jeweils spezifische Vorbehandlungsverfahren und/oder Einfrierverfahren angewendet werden. Bei jeder Fraktion sind andere Verfahrensparameter, insbesondere Vorbehandlungs- und/oder Einfrierparameter, vorgesehen. Für jede Fraktion werden derartige Verfahrensparameter gewählt, dass die Kryokonservierung der Zellverbände optimiert wird, insbesondere mit maximaler Vitalitätserhaltung und/oder Funktionalitätserhaltung erfolgt.

Die Anwendung von Verfahrensparametern der Kryokonservierung umfasst die Einstellung von vorab gewählten Vorbehandlungsparametern und Einfrierparametern. Optimale Vorbehandlungsparameter und Einfrierparameter können durch Testreihen mit Zellverbänden und/oder aus Referenzexperimenten aus der Fachliteratur ermittelt werden. Durch die erfindungsgemäße Wahl von probenspezifischen Verfahrensparametern der Kryokonservierung können vorteilhafterweise die Ausbeute, die Effektivität und/oder die Anwendbarkeit der Kryokonservierung bei verschiedenen Zelltypen verbessert werden.

Nach dem Einfrieren bis zur Kryokonservierungstemperatur werden die gefrorenen Fraktionen vorzugsweise ohne Unterbrechung der Kühlkette zur Lagerung in einer Kryobank bereitgestellt. In der Kryobank erfolgt die Lagerung bei einer Lagerungstemperatur, die von der Kryokonservierungstemperatur abweichen kann.

Vorteilhaftweise sind eine Vielzahl von Eigenschaften verfügbar, auf deren Grundlage die Fraktionierung der Zellverbände erfolgen kann. Gemäß bevorzugten Ausführungsformen der Erfindung erfolgt die Fraktionierung der Zellverbände in Abhängigkeit von mindestens einer der Eigenschaften, die eine Größe, eine Form, eine Masse, eine Elastizität, eine hydraulische Leitfähigkeit, eine Durchlässigkeit für Kryoprotektiva (CPA), eine Resistenz gegenüber Kryoprotektiva, eine chemische Beschaffenheit und eine Zellzusammensetzung der Zellverbände umfassen. Die Fraktionierungseinrichtung ist entsprechend vorzugsweise zur Fraktionierung basierend auf mindestens einer dieser Eigenschaften eingerichtet. Die obigen physikalischen und chemischen Eigenschaften haben sich vorteilhafterweise als besonders gut geeignet für eine effektive Fraktionierung und für die Auswahl optimierter Verfahrensparameter der Kryokonservierung erwiesen.

Die Fraktionierung der Zellverbände kann in Abhängigkeit von mehreren Eigenschaften erfolgen, beispielsweise in Abhängigkeit von der Größe und der CPA-Durchlässigkeit. Bei der Fraktionierung nach mehreren Eigenschaften ist vorzugsweise eine mehrstufige Fraktionierung vorgesehen, bei der in einer ersten Stufe eine erste Eigenschaft, z. B. die Größe der Zellverbände, und in einer mindestens einer weiteren Stufe mindestens eine weitere Eigenschaft, z. B. die CPA-Durchlässigkeit, getestet wird.

Besonders bevorzugt ist eine größenabhängige Fraktionierung vorgesehen. Für eine größenabhängige Trennung sind zahlreiche schonende Trennverfahren verfügbar. Die Wirksamkeit von Verfahrensparametern der Kryokonservierung kann besonders empfindlich von der Größe eines Zellverbandes abhängig sein. Die Größe eines Zellverbandes umfasst z. B. dessen Querschnittsdimension, insbesondere Durchmesser, oder ein anderes charakteristisches geometrisches Maß des Zellverbandes, das sich auf den Stofftransport und/oder den Einfriervorgang auswirkt. Alternativ oder zusätzlich ist die Fraktionierung in Fraktionen mit spezifischen Formen der jeweiligen Zellverbände besonders bevorzugt. Die Form eines Zellverbandes ist die geometrische Gestalt, die der Zellverband im Umgebungsmedium zumindest annähernd aufweist, wie z. B. eine Kugelform oder eine langgestreckte Zylinderform oder eine unregelmäßige Form.

Jede Fraktion wird bei der Kryokonservierung einer Vorbehandlung unterzogen, die sich durch fraktionsspezifisch gewählte Vorbehandlungsparameter auszeichnet. Erfindungsgemäß unterscheiden sich die Vorbehandlungsverfahren für die Fraktionen in Bezug auf mindestens einen der Vorbehandlungsparameter, die eine Dauer, eine Temperatur, einen Druck, eine Medienzusammensetzung, eine Begasungszusammensetzung, Permeabilisierungsbedingungen und eine Medienbewegung der Vorbehandlung umfassen. Diese Vorbehandlungsparameter haben sich vorteilhafterweise als besonders gut geeignet für eine Vorbereitung der Zellverbände für eine effektive Kryokonservierung mit hoher Ausbeute erwiesen.

Vorteilhafterweise können sich die Vorbehandlungsverfahren für die Fraktionen gemäß weiteren Abwandlungen der Erfindung in Bezug auf den Zeitverlauf von mindestens einem der Vorbehandlungsparameter unterscheiden. Die Vorbehandlungsverfahren können sich durch verschiedene Zeitabhängigkeiten des Vorbehandlungsparameters auszeichnen. Mit der Zeitabhängigkeit wird vorteilhafterweise ein zusätzlicher Freiheitsgrad der Optimierung der Vorbehandlung geschaffen.

Erfindungsgemäß ist vorgesehen, dass sich die Einfrierverfahren für die Fraktionen in Bezug auf mindestens einen der Einfrierparameter unterscheiden, die eine Dauer, insbesondere Kühlgeschwindigkeit, eine Temperatur, einen Druck, eine Medienzusammensetzung, eine Begasungszusammensetzung und eine Medienbewegung des Einfrierens umfassen. Die Einfriereinrichtung ist entsprechend vorzugsweise zur Anwendung der Einfrierverfahren mit mindestens einem der genannten Einfrierparameter eingerichtet. Vorteilhafterweise sind damit eine Vielzahl von Parametern verfügbar, die zur Optimierung der Kryokonservierung für verschiedene Fraktionen mit jeweils gleichen Zellverbänden benutzt werden können.

Die Fraktionierung der Zellverbände umfasst eine fluidische Fraktionierung, bei der die Zellverbände in einer fluidischen Umgebung getrennt werden. Daraus ergeben sich weitere Vorteile, da die Zellverbände von deren Bereitstellung, insbesondere Kultivierung, bis zum Einfrieren in einem flüssigen Umgebungsmedium gehalten werden können und ein temporärer Übergang in eine gas- oder dampfförmige Umgebung vermieden wird.

Gemäß einer vorteilhaften Ausführungsform der Erfindung erfolgen die Fraktionierung und Kryokonservierung automatisiert. Die Kryokonservierungsvorrichtung ist für einen automatisierten Betrieb, insbesondere einen Betrieb ohne Eingriff durch einen Betreiber der Kryokonservierungsvorrichtung, eingerichtet. Die Automatisierung bietet Vorteile hinsichtlich der Vermeidung von Verfahrensfehlern, der Reproduzierbarkeit und Genauigkeit der Einstellung von Verfahrensparametern und der Möglichkeit, eine maschinelle Hochdurchsatzfraktionierung in getrennte Fraktionen mit der nachgelagerten Kryokonservierung der getrennten Fraktionen mit hoher Geschwindigkeit und hohem Durchsatz durchzuführen.

Bei der erfindungsgemäßen Fraktionierung, insbesondere Größenfraktionierung, in einer fluidischen Strömung werden die Zellverbände unter der Wirkung von mindestens einem von Strömungskräften der fluidischen Strömung, dielektrophoretischen Kräften in der fluidischen Strömung und Schallwellen in der fluidischen Strömung an verschiedenen Positionen in einem Strömungsprofil der fluidischen Strömung angeordnet. Das Strömungsprofil der fluidischen Strömung umfasst die ortsabhängige Verteilung der Strömungsgeschwindigkeit im Querschnitt der Strömung. Durch die verschiedenen Positionen im Strömungsprofil wird eine Trennung der Zellverbände auf verschiedene Strömungspfade in der Strömung eingeführt. Die Überführung in die verschiedenen Behälter erfolgt durch eine Lenkung der einzelnen Teile des Strömungsprofils über getrennte Teilströme, z. B. Teilkanäle eines fluidischen Systems, und/oder mit verschiedenen Richtungen in die Behälter.

Vorzugsweise ist das fluidische System der Fraktionierungseinrichtung ein fluidisches Mikrosystem, umfassend Kanäle und fluidische Elemente, wie z. B. Verzweigungen oder Kreuzungen, mit charakteristischen Querschnittsdimensionen kleiner als 2 mm. Die fluidische Strömung ist besonders bevorzugt eine parallele, wirbelfreie Strömung, wodurch vorteilhafterweise die Trennung in der Strömung verbessert und die Lenkung der Teile des Strömungsprofils in die Behälter mit einem Abstand von der Trennung der Zellverbände auf die verschiedenen Positionen im Strömungsprofil erfolgen kann.

Die Fraktionierung mittels Strömungskräften der fluidischen Strömung ist eine Größenfraktionierung durch passive Fluidik. Zellverbände ordnen sich entsprechend ihrer Größe an den verschiedenen Positionen im Strömungsprofil an und lassen sich so trennen. Die passive Fluidik hat die folgenden Vorteile. Es handelt sich um ein kontaktloses Verfahren, das die Zellverbände nur wenig belastet und keine Größensensorik erfordert. Die inhomogenen Mischungen von Zellverbänden können portionsweise aufgegeben werden und sich über Laufzeitdifferenzen trennen (chromatographisches Prinzip, Feldflussfraktionierung). Bevorzugt sind jedoch kontinuierliche Verfahren, wie z. B. Pinched Flow Fractionation, PFF), die apparativ einfacher gestaltet werden können und besser skalierbar sind. Beim PFF beispielsweise verlassen Zellverbände unterschiedlicher Größe einen Ausgang, insbesondere Düsenabschnitt, unter unterschiedlichen Winkeln.

Die Fraktionierung mittels dielektrophoretischer Kräfte in der fluidischen Strömung ist eine Größenfraktionierung oder eine Fraktionierung nach einer elektrischen Eigenschaft der Zellverbände durch aktive Fluidik, wie z. B. eine Trennung nach dielektrischen Eigenschaften der Zellverbände, wie z. B. Polarisierbarkeit oder Oberflächenladung. Für diese Trennverfahren ist die Fraktionierungseinrichtung vorzugsweise mit einer Elektrodeneinrichtung ausgestattet, die zur Ausübung dielektrophoretischer Kräfte in einer fluidischen Strömung eingerichtet ist. Die Elektrodeneinrichtung umfasst z. B. mindestens eine Elektrode, die bei Beaufschlagung mit einer Wechselspannung eine dielektrophoretische Feldbarriere erzeugt, die mit der Strömungsrichtung im fluidischen System einen Ablenkwinkel (ungleich 0°) bildet. Die Höhe der dielektrophoretischen Feldbarriere, die auf einen Zellverband wirkt, ist abhängig von der Größe des Zellverbands. Dielektrophoretische Kräfte, die quer zur Strömungsrichtung auf die Zellverbände wirken, werden mit Strömungskräften in der Strömung überlagert. Zellverbände können in Abhängigkeit von ihrer Größe und/oder dielektrischen Eigenschaften und den Strömungskräften die Elektroden an verschiedenen Positionen passieren und entsprechend im Strömungsprofil positioniert werden. Vorteilhafterweise ist auch damit ein kontaktloses Verfahren gegeben, das keine vorgelagerte Sensorik erfordert. Der apparative Aufwand ist jedoch höher als bei der passiven Fluidik.

Alternativ kann die Fraktionierung mittels dielektrophoretischer Kräfte mit einer Sensorik kombiniert werden. Stromaufwärts von der Elektrodeneinrichtung kann eine Sensoreinrichtung angeordnet sein, die zur Erfassung von mindestens einer Eigenschaft der Zellverbände eingerichtet ist. Die Ansteuerung der Elektrodeneinrichtung erfolgt in Abhängigkeit von einem Ausgangssignal der Sensoreinrichtung derart, dass die einzelnen Zellverbände in Abhängigkeit von der erfassten Eigenschaft auf die verschiedenen Positionen im Strömungsprofil gelenkt werden.

Wenn die Fraktionierung mittels Schallwellen in der fluidischen Strömung erfolgt, wird eine weitere Variante der Größenfraktionierung durch aktive Fluidik bereitgestellt. Akustische Felder (stehende und/oder wandernde Schallwellen) geeigneter Frequenz und die Fähigkeit von trägen Körpern, sich an den Feldminima der Schallwellen zu sammeln, werden zur Fraktionierung benutzt. Auch in diesem Fall ist ein kontaktloses Verfahren gegeben, das keine vorgelagerte Sensorik erfordert. Der Größenbereich, auf den sich die akustische Fraktionierung anwenden lässt, ist vorteilhaftweise größer als der für die dielektrophoretische Fraktionierung. Für dieses Trennverfahren ist die Fraktionierungseinrichtung vorzugsweise mit einer Schallquelleneinrichtung ausgestattet, die zur Erzeugung von Schallwellen in der fluidischen Strömung eingerichtet ist.

Gemäß einer weiteren bevorzugten Ausführungsform der Erfindung werden die mindestens eine Eigenschaft der Zellverbände und/oder mindestens eine Zustandsgröße der mindestens zwei Fraktionen sensorisch erfasst. Entsprechend ist die Kryokonservierungsvorrichtung vorzugsweise mit einer Sensoreinrichtung ausgestattet, die zur Erfassung der mindestens einen Eigenschaft der Zellverbände und/oder von mindestens einer Zustandsgröße der mindestens zwei Fraktionen eingerichtet ist. Besonders bevorzugt erfolgen die sensorische Erfassung der mindestens einen Eigenschaft der Zellverbände unmittelbar vor der Fraktionierung und die sensorische Erfassung der mindestens einen Zustandsgröße der Fraktionen unmittelbar vor der Kryokonservierung. Die Sensorik der mindestens einen Eigenschaft der Zellverbände vor der Fraktionierung erweitert vorteilhafterweise die Gruppe von Eigenschaften der Zellverbände, auf deren Grundlage die Fraktionierung erfolgt. Die Sensorik der mindestens einen Zustandsgröße der Fraktionen vor der Kryokonservierung bietet den Vorteil einer weiteren Optimierung der Verfahrensparameter der Kryokonservierung in Abhängigkeit von dem Zustand der Fraktion. Zustandsgrößen der Fraktionen sind z. B. die Dichte oder die Größe der Zellverbände.

Ein weiterer, besonders wichtiger Vorteil der Erfindung für die weitere Nutzung der Zellverbände nach der Kryokonservierung besteht darin, dass auch das vitalitätserhaltende Auftauen fraktionsspezifisch erfolgen kann. Gemäß einer vorteilhaften Variante der Erfindung erfolgt das Auftauen der Zellverbände der mindestens zwei Fraktionen derart, dass für jede Fraktion spezifische Auftauverfahren angewendet werden. Auftauparameter werden wie die Verfahrensparameter der Kryokonservierung für die einzelnen fraktionierten Fraktionen getrennt optimiert, was eine Erhöhung der Vitalitätsrate der aufgetauten Zellverbände erlaubt.

Allgemein können ein Verfahren zum vitalitätserhaltenden Auftauen von mindestens zwei Fraktionen, die durch eine Fraktionierung von Zellverbänden in Abhängigkeit von mindestens einer Eigenschaft der Zellverbände gewonnen und eingefroren wurden, wobei für jede Fraktion ein spezifisches Auftauverfahren angewendet wird, und eine Auftauvorrichtung, die zur Durchführung des Verfahrens konfiguriert ist, als weitere unabhängige Gegenstände der vorliegenden Erfindung betrachtet werden.

Die im Zusammenhang mit dem Verfahren zur Kryokonservierung einer Vielzahl von Zellverbänden aus biologischen Zellen und seinen Ausführungsformen offenbarten Merkmale stellen ebenfalls bevorzugte Merkmale der Kryokonservierungsvorrichtung oder ihrer Ausführungsformen dar. Die oben genannten Aspekte und erfinderischen und bevorzugten Merkmale, insbesondere hinsichtlich des Verfahrens gelten daher auch für die Kryokonservierungsvorrichtung und ihre Komponenten.

Weitere Einzelheiten und Vorteile der Erfindung werden im Folgenden unter Bezug auf die beigefügten Zeichnungen beschrieben. Es zeigen schematisch:
- Figur 1:: einen Ablauf des Verfahrens zur Kryokonservierung einer Vielzahl von Zellverbänden und Komponenten einer Kryokonservierungsvorrichtung mit Merkmalen gemäß bevorzugten Ausführungsformen der Erfindung; und
- Figur 2:: eine Fraktionierungseinrichtung, die zur dielektrophoretischen Trennung von Zellverbänden eingerichtet ist, gemäß einer Ausführungsform der Erfindung.

Merkmale bevorzugter Ausführungsformen der Erfindung werden im Folgenden unter beispielhaftem Bezug auf die Anwendung einer Größenfraktionierung von Zellverbänden beschrieben. Es wird betont, dass die Umsetzung der Erfindung in der Praxis nicht auf eine Größenfraktionierung beschränkt, sondern alternativ oder ergänzend mit einer Fraktionierung möglich ist, die auf einer anderen Eigenschaft der Zellverbände basiert, wie unten mit weiteren Beispielen beschrieben ist. Einzelheiten der Zellverbände und ihrer Bereitstellung und bei konkreten Beispielen angewendete Verfahrensparameter der Kryokonservierung und/oder Auftauparameter werden gewählt, wie aus der Kryokonservierung biologischer Materialien an sich bekannt ist.

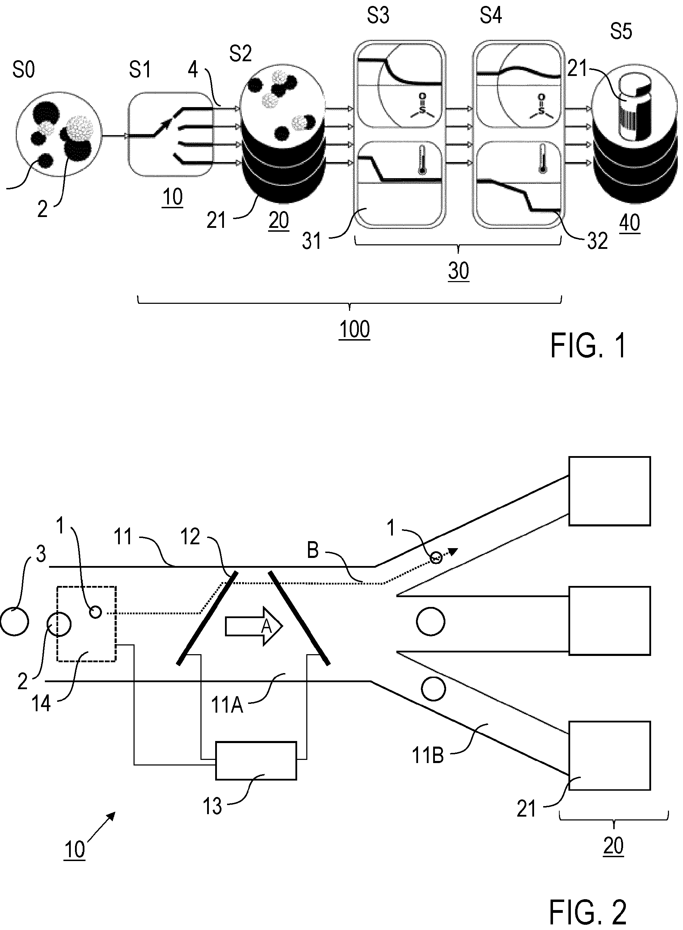

Figur 1 zeigt die Schritte S1 bis S4 des Verfahrens zur Kryokonservierung einer Vielzahl von Zellverbänden 1, 2 und die hierzu verwendete Kryokonservierungsvorrichtung 100 mit einer Fraktionierungseinrichtung 10, einer Behältereinrichtung 20 und einer Einfriereinrichtung 30 gemäß bevorzugten Ausführungsformen der Erfindung. Zusätzlich zeigt Figur 1 einen Schritt S0 der Bereitstellung der Zellverbände 1, 2 und einen Schritt S5 der Einlagerung der eingefrorenen Zellverbände in einer Kryobank 40. Mit dem in Figur 1 gezeigten Beispiel wird z. B. eine automatisierte, fluidische Größenfraktionierung der Zellverbände 1, 2 ausgeführt.

Bei Schritt S0 wird eine inhomogene Probe, z. B. eine Mischung von Zellverbänden 1, 2 verschiedener Größen und/oder eine Mischung von Zellverbänden 1, 2 mit verschiedenen Empfindlichkeiten gegenüber CPA, bereitgestellt. Die Zellverbände 1, 2 umfassen z. B. Organoide, die in an sich bekannter Weise durch eine Kultivierung in einem Nährmedium und mit Differenzierungsfaktoren aus adulten Stammzellen gebildet wurden und z. B. Querschnittsdimensionen im Bereich von 10 µm bis 10 mm oder größer aufweisen. Die Zellverbände 1, 2 werden z. B. in einem Kultivierungsgefäß bereitgestellt.

Bei Schritt S1 werden die Zellverbände 1, 2 mit der schematisch gezeigten Fraktionierungseinrichtung 10 in einzelne Fraktionen 4 (Fraktionen) getrennt, die jeweils Zellverbände mit bestimmten Größen enthalten. Die Fraktionierungseinrichtung 10 ist z. B. aufgebaut, wie unten unter Bezug auf Figur 2 beschrieben ist, und sie wird vorzugsweise automatisiert betrieben. Die Fraktionen 4 werden bei Schritt S2 in Behälter 21 der Behältereinrichtung 20 überführt. Die Behälter 21 umfassen vorzugsweise Kunststoff-Röhrchen mit einem Deckel, insbesondere so genannte PP-Tubes, wie sie bei der nachfolgenden Kryokonservierung bei den Schritten S3 und S4 verwendet werden (siehe Illustration bei Schritt S5). Alternativ können die Behälter andere Aufnahmen, wie z. B. Beutel oder Mikrotiterplatten umfassen. Gemäß einer weiteren Alternative können die Behälter 21 Teil der Inkubationseinheit 31 der Einfriereinrichtung 30 sein. Die einzelnen Fraktionen 4 werden in den für die Lagerung vorgesehenen Behältern 21 gesammelt, bis eine vorbestimmte Beladungsmenge, insbesondere Konzentration (Masse von Zellverbänden pro Volumen des Umgebungsmediums), erreicht ist.

Die Einfriereinrichtung 30 umfasst eine Inkubationseinheit 31 und eine Kühleinheit 32. In der Einfriereinrichtung 30 werden die einzelnen Fraktionen 4 einem der jeweiligen Größe angepassten Vorbehandlungs- und Einfrierprotokoll unterworfen.

In der Inkubationseinheit 31 erfolgt eine Vorbehandlung der Fraktionen. Dies bedeutet, dass für jede Fraktion 4 ein vollständig individuelles Inkubationsprogramm abläuft. Dabei wird mindestens ein CPA (insbesondere Kryoprotektivum) zugeführt, mit dem die Zellverbände beladen werden sollen. Mit zunehmender Größe der Zellverbände werden z. B. zunehmende Konzentrationen von CPA und/oder zunehmende Inkubationszeiten angewendet. Geeignete Kryoprotektiva und deren Konzentrationen können durch Tests ermittelt werden.

Die Inkubation kann des Weiteren eine vorbestimmte Temperierung T(t), Begasung und/oder Perfusion mit vorbestimmten Kryoprotektiva (CPA)-Konzentrationsprofilen C(t, CPA1, CPA2, ...) umfassen. Alternativ oder zusätzlich können temporär membrangängige und/oder sogar toxische CPA zugeführt werden, wenn die Zellverbände diese tolerieren. Weiterhin können eine Eisnukleation (zur Reduktion und Kontrolle der Unterkühlung), eine Medien-Umwälzung (zur Homogenisierung von T und C) und/oder eine Permeabilisierung der Zellverbände mindestens einer Fraktion (zur Beladung mit nicht-membrangängigen CPA) Teil des Vorbehandlungsverfahrens sein. Eine Permeabilisierung kann z. B. chemisch (z.B. mittels DMSO), mit Schallwellen (Sonoporation), mit elektrischen Feldern (Elektroporation), mittels liposomalen Substanzen und/oder durch eine Thermomodulation über Membran-Phasenumwandlung erfolgen. Des Weiteren umfasst die Vorbehandlung in der Inkubationseinheit 31 eine Vorkühlung der Fraktionen 4 auf eine Temperatur oberhalb des Gefrierpunktes der Fraktionen 4.

Die Inkubationseinheit 31 weist vorzugsweise Einzelaufnahmen für die Behälter 21, z. B. Einzelkavitäten auf oder stellt die Behälter durch Reservoire, vorzugsweise für Fraktionen gleicher Volumina, bereit. Die Inkubationseinheit 31 enthält eine Pumpeinrichtung zur Zuführung von CPA (sequentielle Zugabe und/oder Konzentrationserhöhung) und/oder zur Abführung von Medien aus den Behältern. Des Weiteren ist die Inkubationseinheit 31 mit vorzugsweise einem Antrieb, wie z. B. einem Rührwerk, zur Medienbewegung bei der Vorbehandlung in jedem Behälter ausgestattet. Alternativ oder zusätzlich kann eine Vorkühlungseinheit vorgesehen sein, die zur Unterkühlung von Fraktionen eingerichtet ist. Durch die Unterkühlung können Membranänderungen in den Zellen der Zellverbände induziert werden, wodurch die Vorbehandlung, z. B. die Aufnahme von CPAS's beeinflusst werden kann. Alternativ oder zusätzlich kann des Weiteren eine Schallquelle vorgesehen sein, mit der die Zellverbände der Fraktionen einer Ultraschallbehandlung unterzogen werden können. Durch die Ultraschallbehandlung können weitere Membranänderungen in den Zellen der Zellverbände, insbesondere eine Permeabilisierung, induziert werden.

Weitere Vorbehandlungsparameter der größenabhängigen Inkubation umfassen z. B. Konzentrationen der einzelnen CPA, Einwirkdauern der einzelnen CPA, zeitliche Konzentrationsprofile einzelner CPA, angepasste Temperaturverläufe (> 0°C), kontinuierliche Änderungen der Medienzusammensetzung, z. B. mittels Mischeinrichtungen in Verbindung mit der Inkubationseinheit 31 und eines CPA-Reservoirs, und/oder einen Wechsel des Umgebungsmediums (Perfusion).

Anschließend erfolgt das Einfrieren der Fraktionen 4 in der Kühleinheit 32 (Schritt S4). Je nach Eigenschaften der Zellverbände der Fraktionen 4, wie z. B. der Größe oder anderer Eigenschaften, wie die hydraulische Leitfähigkeit der einzelnen Komponenten der Zellverbände, der Anteil von membrangängigen und osmotisch aktiven Zusätzen im Mediums und/oder die Unterkühlung, werden die einzelnen Fraktionen 4 bei verschiedenen Kühlraten und/oder Kühlverläufen kontrolliert eingefroren. Beispielsweise wird eine Kühlrate gleich oder kleiner als -1K/min angewendet. Die Kühlung erfolgt bis zu einer Kryokonservierungstemperatur von z. B. - 80 °C oder darunter, wie z. B. - 140 °C oder darunter.

Temperaturverläufe des Einfrierens einzelner Fraktionen können z. B. gewählt werden, um eine Anpassung an eine Equilibrationsgeschwindigkeit, eine gesteuerte Nukleation zur Verringerung der Unterkühlung, und/oder eine homogene Kühlrate über das Fraktionsvolumen (ggf. mit einem Umwälzen des Mediums und/oder mit Verwendung einer formschlüssigen Passung der Fraktions-Behälter in den Wärmetauscher der Kühleinheit 32) einzustellen.

Die Kühleinheit 32 umfasst für jede Fraktion eine Kühlkammer mit mindestens einem Kühlelement und einem Wärmetauscher. Das Kühlelement ist z. B. ein Peltierelement, ein Stirling-Kühler oder Kühlmitteldurchfluss-Kühler, der beispielweise mit flüssigem Stickstoff oder Isopentan arbeitet. Das Kühlelement ist zur Einstellung einer definierten Kühlrate ausgelegt. Der Wärmetauscher umfasst z. B. eine Aufnahme für den Behälter der jeweiligen Fraktion, vorzugsweise mit Formpassung zwischen dem Behälter und der Aufnahme. Wenn die Behälter 21 Teil der Inkubationseinheit 31 der Einfriereinrichtung 30 sind, erfolgt vor dem Einfrieren ein Transfer in Kryobehälter, wie z. B. die genannten PP-Tubes. Die Kühleinheit 32 kann optional mit einer Nukleationseinrichtung, beispielsweise einer kalten Nadel, ausgestattet sein, mit der eine kontrollierte Nukleation im Behälter induziert wird.

Schließlich werden die Behälter geschlossen und die eingefrorenen Fraktionen bei kryogenen Temperaturen (z. B. - 140 °C) in der Kryobank 40 gelagert (Schritt S5). Der Transfer zur Kryobank 40 erfolgt ohne Unterbrechung der Kühlkette, z. B. mit Hilfe einer gekühlten Schleuse oder durch eine direkte Kopplung der Einfriereinrichtung 30 mit der Kryobank 40.

Zum Auftauen wird der in Figur 1 gezeigte Prozess mit einer Auftaueinrichtung (nicht gezeigt) umgekehrt. Beim Auftauen werden, ähnlich zum größenangepassten Prozess beim Einfrieren, die einzelnen Fraktionen auch während des Auftauens und/oder in einer ersten Recoveryphase bis zum Auswaschen der Hilfsstoffe getrennt in verschiedenen Inkubationseinheiten behandelt. Beispielsweise ist eine größenabhängige Auftaurate, ein größenabhängiges Inkubieren in einem hyperosmolaren Auftaumedium und/oder ein größenabhängiges Auswaschen der getauten Fraktionen vorgesehen. Dadurch kann zum Beispiel der Metabolismus großer Zellverbände durch eine gekühlte Umgebung verlangsamt werden, um eine ausreichende Verdünnung toxischer, membrangängiger CPAs zu gewährleisten, während dies bei kleinen Zellverbänden schneller ablaufen kann.

Nach dem Auftauen kann ein Portionierungsschritt vorgesehen sein, bei dem die getauten Fraktionen einem Vitalitätstest unterzogen und bei erfasster Vitalität in ein vorbestimmtes anwendungsabhängiges Behälterformat, wie z. B. Mikrotiterplatten oder Suspensions-Bioreaktoren, überführt werden. Dabei kann die Größenfraktionierung beibehalten oder aufgegeben werden.

Das Auftauen und/oder die Portionierung können z. B. mit einer Fluidikeinrichtung, insbesondere einem fluidischen Mikrosystem ausgeführt werden.

Figur 2 zeigt beispielhaft eine Fraktionierungseinrichtung 10 in Gestalt einer Fluidikeinrichtung 11, insbesondere eines fluidischen Mikrosystems, mit einem Hauptkanal 11A und Zweigkanälen 11B, die in Richtung des Pfeils A von einer Suspension eines flüssigen Umgebungsmediums mit Zellverbänden 1, 2, 3 verschiedener Größen durchströmt werden. Im Hauptkanal 11A befindet sich eine Elektrodeneinrichtung 12 und eine Sensoreinrichtung 14, die mit einer Steuereinrichtung 13 verbunden sind. Der Hauptkanal 11A verzweigt sich in die Zweigkanäle 11B, die jeweils mit einem der Behälter 21 der Behältereinrichtung 20 verbunden sind.

Die Elektrodeneinrichtung 12 umfasst zwei bandförmige Elektroden oder Elektrodenpaare, z. B. am Boden und/oder einer Deckplatte des Hauptkanal 11A. Bei Beaufschlagung der Elektroden mit Wechselspannungen der Steuereinrichtung 13 kann mit der Elektrodeneinrichtung 12 eine Feldbarriere quer zur Strömung A erzeugt werden. Die Feldbarriere kann passend zu einem mit der Strömung eintreffenden Zellverband temporär erzeugt werden. Durch Zusammenwirken der Feldbarriere mit den Strömungskräften in der Strömung A können Zellverbände auf einen vorbestimmten Strömungspfad gelenkt werden, der zu einem der Zweigkanäle 11B führt (siehe z. B. gepunkteter Strömungspfad B des Zellverbands 1).

Die Sensoreinrichtung 14 ist z. B. ein optischer Sensor, insbesondere eine Kamera in Verbindung mit einer Bildverarbeitungseinrichtung. Mit der Sensoreinrichtung 14 sind die Zellverbände 1, 2, 3 und ihre jeweiligen Größen erfassbar. Informationen über die Positionen und Größen der Zellverbände 1, 2, 3 werden an die Steuereinrichtung 13 geliefert. Die Steuereinrichtung 13 ordnet die die Zellverbände 1, 2, 3 drei vorbestimmten Größen der gewünschten Fraktionen zu und steuert die Elektrodeneinrichtung 12 so an, dass die Zellverbände 1, 2, 3 passend zu den Größen jeweils in einen der Zweigkanäle 11B und über diesen in einen der Behälter 21 gelenkt werden.

Alternativ zu der Ausführungsform in Figur 2 kann die Elektrodeneinrichtung 12 einen dielektrophoretisch wirkenden Feldkäfig umfassen und die Sensoreinrichtung 14 zur Erfassung eines Zellverbandes im Feldkäfig eingerichtet sein. Die Zellverbände werden aufeinanderfolgend im Feldkäfig sensorisch erfasst, in Abhängigkeit von ihren Eigenschaften einer von mehreren Fraktionen zugeordnet und durch Freigabe des Feldkäfigs und ggf. weitere dielektrophoretische Ablenkungen in die jeweilige Fraktion geleitet.

Alternativ oder zusätzlich zur Größenfraktionierung mit dielektrophoretischen Kräften kann mindestens eines der folgenden Trennverfahren zur Fraktionierung vorgesehen sein. Passive Trennverfahren können z. B. eine Fraktionierung im Strömungsprofil (z.B. PFF), eine Dichtefraktionierung (z.B. Sedimentation) und eine geometrische Fraktionierung (z.B. unter Verwendung von Sieben) umfassen. Aktive Trennverfahren können z. B. eine akustische Trennung (z.B. mit stehenden Ultraschallwellen) oder eine optische Trennung (z.B. mit optischen Pinzetten) umfassen.

Alternativ zur optischen Sensorik kann z. B. eine Impedanzmessung an den Zellverbänden vorgesehen sein (z.B. wie bei einem "Coulter Counter"-Gerät) und die Fraktionierung kann in Abhängigkeit vom Ergebnis der Impedanzmessung ausgeführt werden.

Die unter Bezug auf die Figuren 1 und 2 beschriebene Größenfraktionierung kann durch eine Fraktionierung in Bezug auf andere Eigenschaften ergänzt oder ersetzt werden. Beispielsweise kann in einer Fluidikeinrichtung einer Fraktionierungseinrichtung, z. B. in einem Feldkäfig der Fluidikeinrichtung, ein Test der Durchlässigkeit der Zellen von Zellverbänden für Kryoprotektiva und/oder einer Resistenz gegenüber Kryoprotektiva durchgeführt werden. In Abhängigkeit vom Testergebnis können verschiedene Fraktionen gebildet werden, die nachfolgend einer Kryokonservierung mit verschiedenen Verfahrensparametern unterzogen werden. Beispielsweise werden Zellverbände mit einer geringen CPA-Durchlässigkeit der Zellen mit einer längeren CPA-Inkubationsdauer behandelt als Zellverbände mit einer erhöhten CPA-Durchlässigkeit der Zellen.

Die in der vorstehenden Beschreibung, den Zeichnungen und den Ansprüchen offenbarten Merkmale der Erfindung können sowohl einzeln auch in Kombination oder in Unterkombination für die Verwirklichung der Erfindung in ihren verschiedenen Ausgestaltungen von Bedeutung sein.

## Patentansprüche

1. Verfahren zur Kryokonservierung einer Vielzahl von Zellverbänden (1, 2, 3) aus biologischen Zellen, ausgewählt aus in vivo gewachsenen Zell-Matrix-Verbänden, kugelförmigen Zellhaufen und künstlich in vitro gezüchteten Zell-Matrix-Verbänden, mit den Schritten:
- Fraktionierung der Zellverbände (1, 2, 3) in Abhängigkeit von mindestens einer Eigenschaft der Zellverbände (1, 2, 3) in mindestens zwei Fraktionen (4), wobei
die Fraktionierung der Zellverbände (1, 2, 3) eine fluidische Fraktionierung umfasst, bei der die Zellverbände (1, 2, 3) in einer fluidischen Umgebung getrennt werden, und
die Fraktionierung der Zellverbände (1, 2, 3) eine Fraktionierung in einer fluidischen Strömung umfasst, wobei die Zellverbände (1, 2, 3) unter der Wirkung von mindestens einem von dielektrophoretischen Kräften in der fluidischen Strömung und Schallwellen in der fluidischen Strömung an verschiedenen Positionen in einem Strömungsprofil der fluidischen Strömung angeordnet werden,
- Sammlung der Fraktionen (4) in verschiedenen Behältern (21), und
- Kryokonservierung der Zellverbände (1, 2, 3) der mindestens zwei Fraktionen (4), wobei für jede Fraktion jeweils andere spezifische Vorbehandlungsverfahren und/oder Einfrierverfahren angewendet werden, wobei
die Vorbehandlungsverfahren für die Fraktionen (4) sich in Bezug auf mindestens einen der Vorbehandlungsparameter unterscheiden, die eine Dauer, eine Temperatur, einen Druck, eine Medienzusammensetzung, eine Begasungszusammensetzung, Permeabilisierungsbedingungen und eine Medienbewegung der Vorbehandlung umfassen, und
die Einfrierverfahren für die Fraktionen (4) sich in Bezug auf mindestens einen der Einfrierparameter unterscheiden, die eine Dauer, eine Temperatur, einen Druck, eine Medienzusammensetzung, eine Begasungszusammensetzung und eine Medienbewegung des Einfrierens umfassen.

2. Verfahren gemäß Anspruch 1, bei dem
- die Fraktionierung der Zellverbände (1, 2, 3) in Abhängigkeit von mindestens einer der Eigenschaften erfolgt, die eine Größe, eine Form, eine Masse, eine Elastizität, eine hydraulische Leitfähigkeit, eine Durchlässigkeit für Kryoprotektiva (CPA), eine Resistenz gegenüber Kryoprotektiva, eine chemische Beschaffenheit und eine Zellzusammensetzung der Zellverbände (1, 2, 3) umfassen.

3. Verfahren gemäß Anspruch 1 oder 2, bei dem
- die Vorbehandlungsverfahren für die Fraktionen (4) sich in Bezug auf den Zeitverlauf von mindestens einem der Vorbehandlungsparameter unterscheiden.

4. Verfahren gemäß einem der vorhergehenden Ansprüche, mit mindestens einem der folgenden Merkmale :
- die Fraktionen (4) werden in den Behältern (21) gesammelt, in denen nachfolgend die Kryokonservierung erfolgt,
- die eingefrorenen Fraktionen (4) werden ohne Unterbrechung der Kühlkette zur Lagerung in einer Kryobank (40) bereitgestellt, und
- die Fraktionierung und Kryokonservierung erfolgen automatisiert.

5. Verfahren gemäß einem der vorhergehenden Ansprüche, bei dem
- die mindestens eine Eigenschaft der Zellverbände (1, 2, 3) und/oder mindestens eine Zustandsgröße der mindestens zwei Fraktionen (4) sensorisch erfasst werden.

6. Verfahren gemäß einem der vorhergehenden Ansprüche, mit dem weiteren Schritt
- Auftauen der Zellverbände (1, 2, 3) in den mindestens zwei Fraktionen (4), wobei für jede Fraktion spezifische Auftauverfahren angewendet werden.

7. Kryokonservierungsvorrichtung (100), die zur Kryokonservierung einer Vielzahl von Zellverbänden (1, 2, 3) aus biologischen Zellen, ausgewählt aus in vivo gewachsenen Zell-Matrix-Verbänden, kugelförmigen Zellhaufen und künstlich in vitro gezüchteten Zell-Matrix-Verbänden, eingerichtet ist, umfassend:
- eine Fraktionierungseinrichtung (10), die zur Fraktionierung der Zellverbände (1, 2, 3) in Abhängigkeit von mindestens einer Eigenschaft der Zellverbände (1, 2, 3) in mindestens zwei Fraktionen (4) eingerichtet ist, wobei
die Fraktionierungseinrichtung (10) eine Fluidikeinrichtung (11) umfasst, die zur Trennung der Zellverbände (1, 2, 3) in einer fluidischen Umgebung, insbesondere in einer fluidischen Strömung, eingerichtet ist, und
die Fraktionierungseinrichtung (10) eine Elektrodeneinrichtung (12), die zur Ausübung dielektrophoretischer Kräfte in einer fluidischen Strömung eingerichtet ist, und/oder eine Schallquelleneinrichtung umfasst, die zur Erzeugung von Schallwellen in der fluidischen Strömung eingerichtet ist,
- eine Behältereinrichtung (20) mit mindestens zwei verschiedenen Behältern (21), die jeweils zur Sammlung von einer der Fraktionen (4) angeordnet sind, und
- eine Einfriereinrichtung (30), die zur automatisierten Kryokonservierung der Zellverbände (1, 2, 3) in den mindestens zwei Fraktionen (4) eingerichtet ist, wobei die Einfriereinrichtung (30) zur Anwendung von jeweils anderen spezifischen Vorbehandlungsverfahren und/oder Einfrierverfahren für jede der Fraktionen (4) dahingehend eingerichtet ist, dass sie eine Inkubationseinheit (31) zum Vorbehandeln der Fraktionen (4) und eine Kühleinheit (32) zum Einfrieren der Fraktionen (4) umfasst, wobei
die Einfriereinrichtung (30) zur Anwendung der Vorbehandlungsverfahren eingerichtet ist, die sich in Bezug auf mindestens einen der Vorbehandlungsparameter und/oder dessen Zeitverlauf unterscheiden, die eine Dauer, eine Temperatur, einen Druck, eine Medienzusammensetzung, eine Begasungszusammensetzung, Permeabilisierungsbedingungen und eine Medienbewegung bei der Vorbehandlung umfassen, und
die Einfriereinrichtung (30) zur Anwendung der Einfrierverfahren eingerichtet ist, die sich in Bezug auf mindestens einen der Einfrierparameter unterscheiden, die eine Dauer, eine Temperatur, einen Druck, eine Medienzusammensetzung, eine Begasungszusammensetzung und eine Medienbewegung beim Einfrieren umfassen.

8. Kryokonservierungsvorrichtung gemäß Anspruch 7, bei der
- die Fraktionierungseinrichtung (10) zur Fraktionierung der Zellverbände (1, 2, 3) in Abhängigkeit von mindestens einer der Eigenschaften eingerichtet ist, die eine Größe, eine Form, eine Masse, eine Elastizität, eine hydraulische Leitfähigkeit, eine Durchlässigkeit für Kryoprotektiva (CPA), eine Resistenz gegenüber Kryoprotektiva, eine chemische Beschaffenheit und eine Zellzusammensetzung der Zellverbände (1, 2, 3) umfassen.

9. Kryokonservierungsvorrichtung gemäß Anspruch 7 oder 8, mit mindestens einem der folgenden Merkmale :
- die mindestens zwei Behälter (21) sind Teil der Einfriereinrichtung (30), und
- die Kryokonservierungsvorrichtung (100) ist für einen automatisierten Betrieb eingerichtet.

10. Kryokonservierungsvorrichtung gemäß einem der Ansprüche 7 bis 9, umfassend
- eine Sensoreinrichtung (14), die zur Erfassung der mindestens einen Eigenschaft der Zellverbände (1, 2, 3) und/oder von mindestens einer Zustandsgröße der mindestens zwei Fraktionen (4) eingerichtet ist.

## Claims

1. A method for cryopreserving a plurality of cell clusters (1, 2, 3) of biological cells selected from cell-matrix clusters grown in vivo, spherical clusters of cells and cell-matrix clusters grown artificially in vitro, comprising the steps of:
- fractionating the cell clusters (1, 2, 3) into at least two fractions (4) in dependency on at least one property of the cell clusters (1, 2, 3), wherein
the fractionation of the cell clusters (1, 2, 3) comprises fluidic fractionation, in which the cell clusters (1, 2, 3) are separated in a fluid environment, and
the fractionation of the cell clusters (1, 2, 3) comprises fractionation in a fluid flow, wherein the cell clusters (1, 2, 3) are arranged at different positions in a flow profile of the fluid flow under the effect of at least one of dielectrophoretic forces in the fluid flow, and sound waves in the fluid flow,
- collecting the fractions (4) in different containers (21), and
- cryopreserving the cell clusters (1, 2, 3) of the at least two fractions (4), wherein specific pretreatment methods and/or freezing methods are used for each fraction, wherein
the pretreatment methods for the fractions (4) differ in terms of at least one of the pretreatment parameters including a duration, a temperature, a pressure, a medium composition, a gas supply composition, permeabilisation conditions and a movement of the medium of the pretreatment and
the freezing methods for the fractions (4) differ in terms of at least one of the freezing parameters including a duration, a temperature, a pressure, a medium composition, a gas supply composition, and a movement of the medium of the freezing.

2. The method according to claim 1, wherein
- the fractionation of the cell clusters (1, 2, 3) takes place on the basis of at least one of the properties including a size, a shape, a mass, an elasticity, a hydraulic conductivity, a cryoprotectant (CPA) permeability, a resistance to cryoprotectants, a chemical constitution and a cell composition of the cell clusters (1, 2, 3).

3. The method according to claim 1 or 2, wherein
- the pretreatment methods for the fractions (4) differ in terms of the time profile of at least one of the pretreatment parameters.

4. The method according to any one of the preceding claims, comprising at least one of the following features:
- the fractions (4) are collected in the containers (21) in which the cryopreservation subsequently takes place,
- the frozen fractions (4) are provided for storage in a cryobank (40) without interrupting the cold chain, and
- the fractionation and cryopreservation are carried out in an automated manner.

5. The method according to any one of the preceding claims, wherein
- the at least one property of the cell clusters (1, 2, 3) and/or at least one state variable of the at least two fractions (4) are detected by means of sensing.

6. The method according to any one of the preceding claims, comprising the additional step of
- thawing the cell clusters (1, 2, 3) in the at least two fractions (4), wherein specific thawing methods are used for each fraction.

7. A cryopreservation apparatus (100), which is adapted for cryopreserving a plurality of cell clusters (1, 2, 3) of biological cells selected from cell-matrix clusters grown in vivo, spherical clusters of cells and cell-matrix clusters grown artificially in vitro, comprising:
- a fractionation device (10), which is adapted for fractionating the cell clusters (1, 2, 3) into at least two fractions (4) in dependency on at least one property of the cell clusters (1, 2, 3), wherein the fractionation device (10) comprises a fluidics device (11) which is adapted for separating the cell clusters (1, 2, 3) in a fluid environment, in particular in a fluid flow, and
the fractionation device (10) comprises an electrode device (12) which is adapted to apply dielectrophoretic forces in a fluid flow, and/or comprises a sound source device which is adapted to generate sound waves in the fluid flow,
- a container device (20) having at least two different containers (21), each of which is arranged for collecting one of the fractions (4), and
- a freezing device (30), which is adapted for automated cryopreserving the cell clusters (1, 2, 3) in the at least two fractions (4), wherein the freezing device (30) is adapted for using specific pretreatment methods and/or freezing methods for each of the fractions (4) such that it comprises an incubation unit (31) for pretreating the fractions (4) and a cooling unit (32) for freezing the fractions (4), wherein
the freezing device (30) is adapted to apply the pretreatment methods which differ in terms of at least one of the pretreatment parameters and/or the time profile of said parameter, said parameters including a duration, a temperature, a pressure, a medium composition, a gas supply composition, permeabilisation conditions and a movement of the medium during the pretreatment, and the freezing device (30) is adapted to apply the freezing methods which differ in terms of at least one of the freezing parameters, said parameters including a duration, a temperature, a pressure, a medium composition, a gas supply composition, and a movement of the medium during the freezing.

8. The cryopreservation apparatus according to claim 7, wherein
- the fractionation device (10) is adapted for fractionating the cell clusters (1, 2, 3) on the basis of at least one of the properties including a size, a shape, a mass, an elasticity, a hydraulic conductivity, a cryoprotectant (CPA) permeability, a resistance to cryoprotectants, a chemical constitution and a cell composition of the cell clusters (1, 2, 3).

9. The cryopreservation apparatus according to claim 7 or 8, comprising at least one of the following features:
- the at least two containers (21) are part of the freezing device (30), and
- the cryopreservation apparatus (100) is adapted for automated operation.

10. The cryopreservation apparatus according to any one of claims 7 to 9, comprising
- a sensor device (14) which is adapted for detecting the at least one property of the cell clusters (1, 2, 3) and/or at least one state variable of the at least two fractions (4).

## Revendications

1. Procédé de cryoconservation d'une multitude de groupements cellulaires (1, 2, 3) constitués par des cellules biologiques, choisis parmi des groupements cellulaires matriciels cultivés in vivo, des amas cellulaires sphériques et des groupements cellulaires matriciels cultivés artificiellement in vitro, comprenant les étapes de :
- fractionnement des groupements cellulaires (1, 2, 3) en fonction d'au moins une propriété des groupements cellulaires (1, 2, 3) en au moins deux fractions (4),
le fractionnement des groupements cellulaires (1, 2, 3) comprenant un fractionnement fluidique lors duquel les groupements cellulaires (1, 2, 3) sont séparés dans un environnement fluidique et
le fractionnement des groupements cellulaires (1, 2, 3) comprenant un fractionnement dans un écoulement fluidique, les groupements cellulaires (1, 2, 3) étant agencés en différentes positions dans un profil d'écoulement de l'écoulement fluidique sous l'action d'au moins un élément parmi des forces diélectrophorétiques dans l'écoulement fluidique et des ondes sonores dans l'écoulement fluidique,
- collecte des fractions (4) dans différents récipients (21) et
- cryoconservation des groupements cellulaires (1, 2, 3) desdites au moins deux fractions (4), des procédés spécifiques différents de prétraitement et/ou de congélation étant à chaque fois utilisés pour chaque fraction,
les procédés de prétraitement des fractions (4) se distinguant en ce qui concerne au moins l'un des paramètres de prétraitement, qui comprennent une durée, une température, une pression, une composition de milieu, une composition de gazage, des conditions de perméabilisation et une agitation du milieu du prétraitement et
les procédés de congélation des fractions (4) de distinguant en ce qui concerne au moins l'un des paramètres de congélation, qui comprennent une durée, une température, une pression, une composition de milieu, une composition de gazage et une agitation du milieu de la congélation.

2. Procédé selon la revendication 1, dans lequel
- le fractionnement des groupements cellulaires (1, 2, 3) est effectué en fonction d'au moins l'une des propriétés, qui comprennent une grosseur, une forme, une masse, une élasticité, une conductivité hydraulique, une perméabilité aux agents cryoprotecteurs (CPA), une résistance aux agents cryoprotecteurs, une nature chimique et une composition cellulaire des groupements cellulaires (1, 2, 3).

3. Procédé selon la revendication 1 ou 2, dans lequel
- les procédés de prétraitement des fractions (4) se distinguent en ce qui concerne la ligne temporelle d'au moins l'un des paramètres de prétraitement.

4. Procédé selon l'une des revendications précédentes, présentant au moins l'une des caractéristiques suivantes :
- les fractions (4) sont collectées dans les récipients (21), dans lesquels la cryoconservation est ensuite effectuée,
- les fractions congelées (4) sont mises à disposition sans interruption de la chaîne de froid pour le stockage dans une banque cryogénique (40) et
- le fractionnement et la cryoconservation sont effectués de manière automatisée.

5. Procédé selon l'une des revendications précédentes, dans lequel
- ladite au moins une propriété des groupements cellulaires (1, 2, 3) et/ou au moins une grandeur d'état desdites au moins deux fractions (4) est/sont détectée(s) par des capteurs.

6. Procédé selon l'une des revendications précédentes, comportant l'étape supplémentaire de
- décongélation des groupements cellulaires (1, 2, 3) dans lesdites au moins deux fractions (4), des procédés de décongélation spécifiques étant utilisés pour chaque fraction.

7. Appareil de cryoconservation (100) conçu pour la cryoconservation d'une multitude de groupements cellulaires (1, 2, 3) constitués par des cellules biologiques, choisis parmi des groupements cellulaires matriciels cultivés in vivo, des groupements cellulaires sphériques et des groupements cellulaires matriciels cultivés artificiellement in vitro, comprenant :
- un dispositif de fractionnement (10) qui est conçu pour la fractionnement des groupements cellulaires (1, 2, 3) en fonction d'au moins une propriété des groupements cellulaires (1, 2, 3) en au moins deux fractions (4),
le dispositif de fractionnement (10) comprenant un dispositif fluidique (11) conçu pour séparer les groupements cellulaires (1, 2, 3) dans un environnement fluidique, en particulier dans un écoulement fluidique, et
le dispositif de fractionnement (10) comprenant un dispositif d'électrode (12), qui est conçu pour exercer des forces diélectrophorétiques dans un écoulement fluidique, et/ou un dispositif de source sonore, qui est conçu pour générer des ondes sonores dans l'écoulement fluidique,
- un dispositif (20) à récipients présentant au moins deux récipients (21) différents, chacun étant agencé pour la collecte de l'une des fractions (4) et
- un dispositif de congélation (30) qui est conçu pour la cryoconservation automatisée des groupements cellulaires (1, 2, 3) dans lesdites au moins deux fractions (4), le dispositif de congélation (30) étant conçu pour l'application de procédés de prétraitement et/ou de congélation spécifiques à chaque fois différents pour chacune des fractions (4), en ce sens qu'il comprend une unité d'incubation (31) pour le prétraitement des fractions (4) et une unité de refroidissement (32) pour la congélation des fractions (4),
le dispositif de congélation (30) étant conçu pour appliquer des procédés de prétraitement, qui se distinguent en ce qui concerne au moins l'un des paramètres de prétraitement et/ou leur ligne temporelle, qui comprennent une durée, une température, une pression, une composition de milieu, une composition de gazage, des conditions de perméabilisation et une agitation du milieu lors du prétraitement et
le dispositif de congélation (30) étant conçu pour appliquer les procédés de congélation, qui se distinguent en ce qui concerne au moins l'un des paramètres de congélation, qui comprennent une durée, une température, une pression, une composition de milieu, composition de gazage et une agitation du milieu lors de la congélation.

8. Appareil de cryoconservation selon la revendication 7, dans lequel
- le dispositif de fractionnement (10) est conçu pour le fractionnement des groupements cellulaires (1, 2, 3) en fonction d'au moins l'une des propriétés, qui comprennent une grosseur, une forme, une masse, une élasticité, une conductivité hydraulique, une perméabilité aux agents cryoprotecteurs (CPA), une résistance aux agents cryoprotecteurs, une nature chimique et une composition cellulaire des groupements cellulaires (1, 2, 3).

9. Appareil de cryoconservation selon la revendication 7 ou 8, présentant au moins l'une des caractéristiques suivantes :
- lesdits au moins deux récipients (21) font partie du dispositif de congélation (30) et
- l'appareil de cryoconservation (100) est conçu pour fonctionner de manière automatisée.

10. Appareil de cryoconservation selon l'une des revendications 7 à 9, comprenant
- un dispositif (14) à capteurs est conçu pour la détection de ladite au moins une propriété des groupements cellulaire (1, 2, 3) et/ou d'au moins une grandeur d'état desdites au moins deux fractions (4).
